(19) Europäisches Patentamt
European Patent Office
Office européen des brevets

(11) EP 4 769 622 A1

(12) # EUROPEAN PATENT APPLICATION

(43) Date of publication:
**01.07.2026 Bulletin 2026/27**

(21) Application number: **25226249.8**

(22) Date of filing: **22.12.2025**

(51) International Patent Classification (IPC):
***H01M 10/0567*** *(2010.01)* ***C07D 307/00*** *(2006.01)*

(52) Cooperative Patent Classification (CPC):
**H01M 10/0567; C07D 307/33;** H01M 10/0525

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **27.12.2024 KR 20240199294**

(71) Applicants:
- **SK On Co., Ltd.**
  **Seoul 03161 (KR)**
- **SK Innovation Co., Ltd.**
  **Seoul 03188 (KR)**

(72) Inventors:
- **LEE, Hyun Ho**
  **34124 Daejeon (KR)**
- **JEONG, Myung Hwan**
  **34124 Daejeon (KR)**
- **KIM, Myoung Lae**
  **34124 Daejeon (KR)**

(74) Representative: **Prüfer & Partner mbB**
**Patentanwälte · Rechtsanwälte**
**Sohnckestraße 12**
**81479 München (DE)**

(54) **ELECTROLYTE FOR LITHIUM SECONDARY BATTERY AND LITHIUM SECONDARY BATTERY INCLUDING THE SAME**

(57) An electrolyte for a lithium secondary battery and a lithium secondary battery are provided. The electrolyte for a lithium secondary battery includes a butyrolactone-based compound including at least one alkenyl group, and a lithium salt. The lithium secondary battery includes an electrode assembly including a cathode and an anode, and the electrolyte for a lithium secondary battery.

FIG. 1

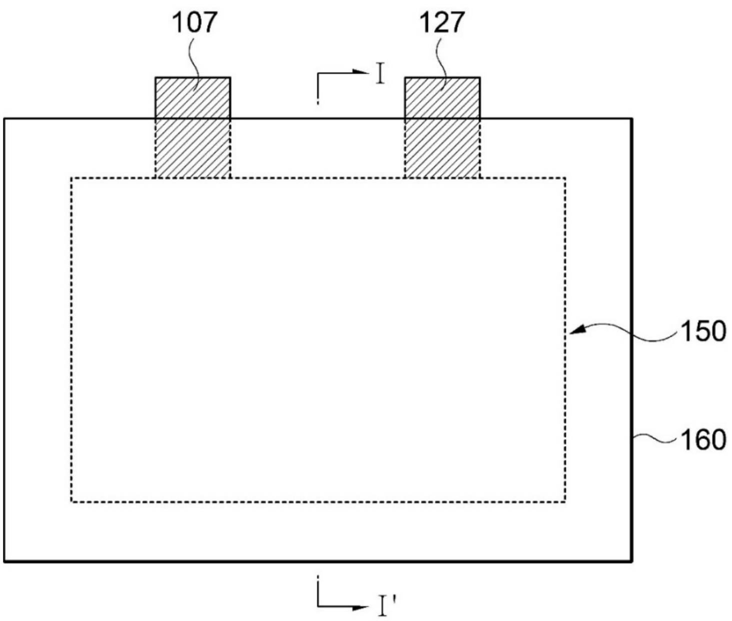




Processed by Luminess, 75001 PARIS (FR)

## Description

The present disclosure relates to batteries such as lithium secondary batteries.

BACKGROUND

A secondary rechargeable battery which can be charged and discharged repeatedly has been widely employed as a power source for mobile electronic devices such as camcorders, mobile phones, laptop computers, etc.. Recently, battery packs including the secondary batteries are being developed and applied as power sources for eco-friendly vehicles such as fully battery powered electric vehicles and hybrid automobiles.

SUMMARY

The present disclosure relates to technical features of secondary batteries, including the composition of an electrolyte for a lithium secondary battery that includes a butyrolactone-based compound and an electrolyte salt.

The technical features of the present disclosure can be implemented to, in various applications, provide an electrolyte having improved properties at high temperature.

The technical features of the present disclosure can also be implemented to provide a lithium secondary battery having improved electrochemical energy storage properties at high temperature.

An electrolyte for a lithium secondary battery according to exemplary embodiments of the present disclosure may include a butyrolactone-based compound having at least one alkenyl group, and a lithium salt.

In some embodiments, the butyrolactone-based compound may include a gamma-butyrolactone-based compound.

In some embodiments, the butyrolactone-based compound may further include at least one alkyl group.

In some embodiments, the butyrolactone-based compound may be represented by Chemical Formula 1.

[Chemical Formula 1]

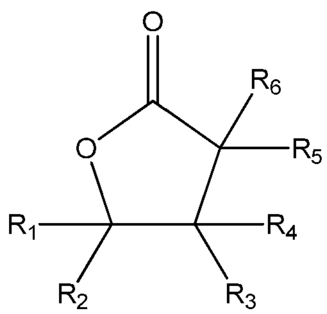

In Chemical Formula 1, $R_1$ to $R_6$ may each independently be hydrogen, a $C_1$-$C_{10}$ substituted or unsubstituted alkyl group, or a $C_2$-$C_{10}$ substituted or unsubstituted alkenyl group, and at least one of $R_1$ to $R_6$ may be a $C_2$-$C_{10}$ substituted or unsubstituted alkenyl group.

In some embodiments, at least one of $R_1$ to $R_6$ may be a $C_1$-$C_6$ substituted or unsubstituted alkyl group.

In some embodiments, one or two of $R_1$ to $R_6$ may be a substituted or unsubstituted $C_1$-$C_6$ alkyl group, and one of remaining groups may be a substituted or unsubstituted $C_2$-$C_{10}$ alkenyl group.

In some embodiments, $R_1$ to $R_6$ may each independently be hydrogen, a substituted or unsubstituted $C_1$-$C_6$ alkyl group, or a substituted or unsubstituted $C_2$-$C_6$ alkenyl group, and at least one of $R_1$ to $R_6$ may be a substituted or unsubstituted $C_2$-$C_6$ alkenyl group.

In some embodiments, $R_1$ to $R_6$ may each independently be hydrogen, a $C_1$-$C_4$ substituted or unsubstituted alkyl group, or a $C_2$-$C_4$ substituted or unsubstituted alkenyl group, and at least one of $R_1$ to $R_6$ may be a $C_2$-$C_4$ substituted or unsubstituted alkenyl group.

In some embodiments, at least one of $R_1$ and $R_2$ may be a $C_2$-$C_{10}$ substituted or unsubstituted alkenyl group.

In some embodiments, one of $R_1$ and $R_2$ may be a $C_1$-$C_{10}$ substituted or unsubstituted alkyl group, and the other may be a $C_2$-$C_{10}$ substituted or unsubstituted alkenyl group.

In some embodiments, the butyrolactone-based compound may include a compound represented by any one of Chemical Formulae 2-1 to 2-3.

[Chemical Formula 2-1]

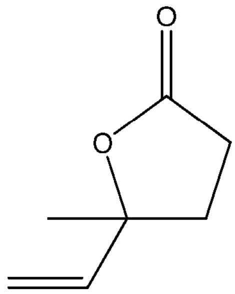

[Chemical Formula 2-2]

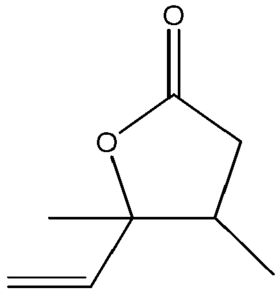

[Chemical Formula 2-3]

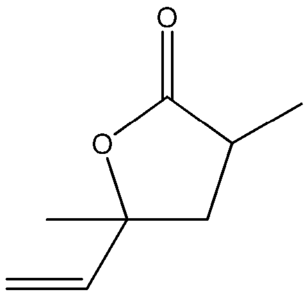

In some embodiments, a content of the butyrolactone-based compound may be greater than 0 wt%, and 10 wt% or less based on a total weight of the electrolyte.

In some embodiments, the electrolyte for a lithium secondary battery may further include an organic solvent. The organic solvent may include at least one selected from a carbonate solvent, an ester solvent, an ether solvent, a ketone solvent, an alcohol solvent or an aprotic solvent.

In some embodiments, the electrolyte for a lithium secondary battery may further include at least one auxiliary additive selected from a cyclic carbonate compound, a fluorine-containing carbonate compound, a lithium phosphate compound, a sultone compound, a borate compound, a sulfate compound or a sulfite compound.

In some embodiments, a content of the auxiliary additive may be in a range from 0.01 wt% to 5 wt% based on a total weight of the electrolyte.

A lithium secondary battery may include an electrode assembly including a cathode and an anode, and the above-described electrolyte for a lithium secondary battery.

In some embodiments, the cathode may include a cathode active material that includes lithium metal oxide particles having a single particle structure.

In some embodiments, a content of nickel in the lithium metal oxide particles may be in a range from 50 mol% to 70 mol% based on total moles of elements excluding lithium and oxygen contained in the lithium metal oxide particles.

In some embodiments, the lithium secondary battery may have an operating voltage of 4.35 V to 4.5 V.

In some embodiments, the anode may include a solid electrolyte interphase (SEI) film on a surface thereof, and the SEI film may be formed from the electrolyte for a lithium secondary battery.

The lithium secondary battery according to some examplary embodiments may include an additive in an

electrolyte to improve high-temperature storage performance in a high-voltage environment.

According to examplary embodiments, even following storage at high temperature, a resistance increase ratio and a thickness increase ratio of the lithium secondary battery may be reduced during high voltage operation. Accordingly, energy efficiency, stability and life-span properties of the lithium secondary battery may be improved.

The electrolyte for a lithium secondary battery and the lithium secondary battery according to the present disclosure may be widely applied in green technology fields using batteries, such as electric vehicles, battery charging stations, solar power generation, wind power generation, etc.. The lithium secondary battery according to the present disclosure may be used for eco-friendly electric vehicles and hybrid vehicles to prevent climate change by suppressing air pollution and/or greenhouse gas emissions. etc.

BRIEF DESCRIPTION OF THE DRAWINGS

FIGS. 1 and 2 are a schematic plan view and a schematic cross-sectional view, respectively, illustrating a lithium secondary battery in accordance with example embodiments.

DETAILED DESCRIPTION

Examples of the secondary battery include a lithium secondary battery, a sodium secondary battery, a potassium secondary battery, a nickel-cadmium battery, a nickel-hydrogen battery, etc. The lithium secondary battery among the secondary batteries is being actively developed due to high operational voltage and energy density per unit weight, a high charging rate, a compact dimension, etc. that are suitable for a wide range of applications.

The lithium secondary battery may include an electrode assembly including a cathode, an anode and a separation layer (separator), and an electrolyte immersing the electrode assembly. The lithium secondary battery may further include an outer case having, e.g., a pouch shape for accommodating the electrode assembly and the electrolyte.

As the range of applications for lithium secondary batteries expands, extended life-span, higher capacity and improved operation stability are required. Accordingly, lithium secondary batteries providing uniform power and capacity even when charging and discharging are repeated are being developed.

However, as charging and discharging are repeated, power and capacity may be decreased due to, e.g., damage to the surface of nickel-based lithium metal oxide particles used as a cathode active material, and side reactions between the nickel-based lithium metal oxide and an electrolyte. Additionally, stability of the battery may deteriorate in harsh environments such as at high or low temperature.

The disclosed technology can be implemented, in various applications, to an electrolyte for a lithium secondary battery to achieve high-voltage, high-temperature storage performance with stability.

According to examplary embodiments, an electrolyte for a lithium secondary battery may include a butyrolactone-based compound including at least one alkenyl group, and a lithium salt. The electrolyte may be a liquid electrolyte or a semi-solid electrolyte, and the semi-solid electrolyte may include a gel-polymer electrolyte.

In some embodiments, the butyrolactone-based compound may be present as an additive to the electrolyte.

In some embodiments, the additive may be present in a concentration greater than 0 wt%, and 10 wt% or less based on a total weight of the electrolyte.

In examplary embodiments, a lithium secondary battery may include an electrode assembly including a cathode and an anode, and the electrolyte for a lithium secondary battery. For example, the electrode assembly may include cathodes and anodes that are repeatedly stacked, and the electrolyte may impregnate the electrode assembly.

In some embodiments, the anode may include a solid electrolyte interphase (SEI) film on a surface thereof. The solid electrolyte interphase film may be generated from the electrolyte for a lithium secondary battery. The solid electrolyte interphase film may include a moiety derived from a compound in the electrolyte. For example, the solid electrolyte interfacial film may include a functional group derived from the additive. Accordingly, high-voltage, high-temperature storage performance of the lithium secondary battery may be improved.

As used herein, "high voltage" may refer to a driving or operating voltage of the battery in a range from 4.3 V to 4.7 V, or from 4.35 V to 4.5 V.

As used herein, "A-based compound" may refer to a compound including a functional group A or a derivative thereof.

The term "substituted or unsubstituted" as used herein refers to, e.g., a substitution with at least one group selected from a deuterium atom, a halogen atom, a cyano group, a nitro group, an amino group, a silyl group, an oxy group, a thio group, a sulfinyl group, a sulfonyl group, a carbonyl group, an ester group, boron, a phosphine oxide group, a phosphine sulfide group, an alkyl group (e.g., a $C_1$-$C_{60}$, $C_1$-$C_{10}$ alkyl group), an alkenyl group (e.g., a $C_2$-$C_{60}$, $C_2$-$C_{10}$ alkenyl group), an alkynyl group (e.g., a $C_2$-$C_{60}$, $C_2$-$C_{10}$ alkynyl group), an alkoxy group (e.g., a $C_1$-$C_{60}$, $C_1$-$C_{10}$ alkoxy group), a hydrocarbon ring group, an aryl group (e.g., a $C_6$-$C_{60}$ aryl group), a heterocyclic group (e.g., a $C_1$-$C_{60}$

heterocyclic group), or non-substitution by the above group. For example, a "substituted alkyl group" may refer to an alkyl group in which at least one of hydrogen atoms is substituted with the above-mentioned substituent, and thus the substituent is further bonded to a carbon atom of the alkyl group.

The substituent may include a combination selected from the groups described above. For example, at least one of hydrogen atoms of an alkyl group, an aryl group, etc., included as the substituent may be substituted with a deuterium atom, a halogen atom, a cyano group, a nitro group, an amino group, a silyl group, an oxy group, a thio group, a sulfinyl group, a sulfonyl group, a carbonyl group, an ester group, boron, a phosphine oxide group, a phosphine sulfide group, an alkyl group, an alkenyl group, an alkynyl group, a hydrocarbon ring group, an aryl group, a heterocyclic group, or other groups.

Among the above substituents, a multivalent substituent such as an amino group, a phosphine sulfide group, a phosphine oxide group, a sulfinyl group, a sulfonyl group, an oxy group, a carbonyl group, an ester group, etc., may be substituted with a $C_1$-$C_{10}$ alkyl group, a $C_1$-$C_{10}$ alkenyl group, a $C_1$-$C_{10}$ alkynyl group, or a $C_6$-$C_{10}$ aryl group.

In the term "substituted or unsubstituted $C_a$-$C_b$ Y group" used herein, $C_a$-$C_b$ refers to the carbon number of the Y group in a unsubstituted state, and may not include the carbon number of the substituent. A

The alkyl group refers to a monovalent hydrocarbon group from which one hydrogen atom is removed from a straight-chain or branched-chain hydrocarbon group. For example, the alkyl group may include a methyl group, an ethyl group, a propyl group, a sec-butyl group, a tert-butyl group, an iso-butyl group, a pentyl group, a neopentyl group, a 2-ethylbutyl group, a 3,3-dimethylbutyl group, a hexyl group, a heptyl group, an octyl group, etc.

The alkenyl group refers to a monovalent unsaturated hydrocarbon group from which one hydrogen atom removed from a straight-chain or branched-chain hydrocarbon group and in which a carbon-carbon double bond is included. For example, the alkenyl group may include an ethenyl group (vinyl group), a propenyl group, a 1-butenyl group, a 2-butenyl group, a pentenyl group, a neopentenyl group, a hexenyl group, a 2-methylpropenyl group, a 3-methylbutenyl group, a 3,3-dimethylpentenyl group, etc.

An electrolyte for a lithium secondary battery according to embodiments may include a butyrolactone-based compound including at least one alkenyl group.

In some embodiments, the alkenyl group may be a substituted or unsubstituted $C_2$-$C_{10}$ alkenyl group, a substituted or unsubstituted $C_2$-$C_6$ alkenyl group, or a substituted or unsubstituted $C_2$-$C_4$ alkenyl group.

For example, the butyrolactone-based compound including at least one alkenyl group may be used so that the reactivity of the alkenyl group may readily form a solid electrolyte interphase (SEI) film on an electrode active material during high-voltage charge/discharge. Further, a film containing an alkenyl-based double bond may be formed so that an anode may be stably operated under high voltage.

For example, the solid electrolyte interphase film may be formed on a surface of the anode.

In some embodiments, the butyrolactone-based compound may include a gamma-butyrolactone compound.

In some embodiments, the butyrolactone-based compound may further include at least one alkyl group. The alkyl group may be a substituted or unsubstituted $C_1$-$C_{10}$ alkyl group, a substituted or unsubstituted $C_1$-$C_6$ alkyl group, a substituted or unsubstituted $C_1$-$C_4$ alkyl group, a substituted or unsubstituted $C_1$ or $C_2$ alkyl group, or a substituted or unsubstituted methyl group.

In some embodiments, the butyrolactone-based compound may be represented by Chemical Formula 1.

[Chemical Formula 1]

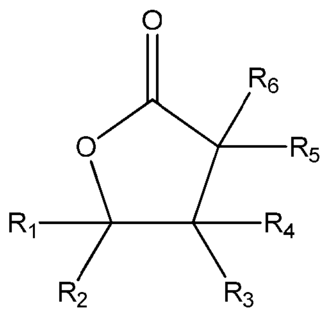

In Chemical Formula 1, $R_1$ to $R_6$ may each independently be hydrogen, a $C_1$-$C_{10}$ substituted or unsubstituted alkyl group, or a $C_2$-$C_{10}$ substituted or unsubstituted alkenyl group, and at least one of $R_1$ to $R_6$ may be a $C_2$-$C_{10}$ substituted or unsubstituted alkenyl group.

In some embodiments, the $C_1$-$C_{10}$ substituted or unsubstituted alkyl group of $R_1$ to $R_6$ may be a $C_1$-$C_6$ substituted or unsubstituted alkyl group, a $C_1$-$C_4$ substituted or unsubstituted alkyl group, a $C_1$ or $C_2$ substituted or unsubstituted alkyl group, or a substituted or unsubstituted methyl group.

In some embodiments, the $C_2$-$C_{10}$ substituted or unsubstituted alkenyl group of $R_1$ to $R_6$ may be a $C_2$-$C_6$ substituted or unsubstituted alkenyl group, a $C_2$-$C_4$ substituted or unsubstituted alkenyl group, or a substituted or unsubstituted ethenyl group.

In some embodiments, at least one of $R_1$ to $R_6$ may be a substituted or unsubstituted $C_1$-$C_6$ alkyl group.

In some embodiments, one or two of $R_1$ to $R_6$ may be a substituted or unsubstituted $C_1$-$C_6$ alkyl group.

In some embodiments, $R_1$ to $R_6$ may each independently be hydrogen, a substituted or unsubstituted $C_1$-$C_{10}$ alkyl group, or a substituted or unsubstituted $C_2$-$C_{10}$ alkenyl group, and one or two of $R_1$ to $R_6$ may be a substituted or unsubstituted $C_1$-$C_6$ alkyl group, and one of the others may be a substituted or unsubstituted $C_2$-$C_{10}$ alkenyl group.

In some embodiments, $R_1$ to $R_6$ may each independently be hydrogen, a substituted or unsubstituted $C_1$-$C_6$ alkyl group, or a substituted or unsubstituted $C_2$-$C_6$ alkenyl group, and at least one of $R_1$ to $R_6$ may be a substituted or unsubstituted $C_2$-$C_6$ alkenyl group.

In some embodiments, $R_1$ to $R_6$ may each independently be hydrogen, a $C_1$-$C_4$ substituted or unsubstituted alkyl group, or a $C_2$-$C_4$ substituted or unsubstituted alkenyl group, and at least one of $R_1$ to $R_6$ may be a $C_2$-$C_4$ substituted or unsubstituted alkenyl group.

In some embodiments, at least one of $R_1$ and $R_2$ may be a $C_2$-$C_{10}$ substituted or unsubstituted alkenyl group.

In some embodiments, one of $R_1$ and $R_2$ may be a $C_1$-$C_{10}$ substituted or unsubstituted alkyl group, and the other may be a $C_2$-$C_{10}$ substituted or unsubstituted alkenyl group.

In some embodiments, one of $R_1$ and $R_2$ may be a $C_1$-$C_6$ substituted or unsubstituted alkyl group, and the other may be a $C_2$-$C_6$ substituted or unsubstituted alkenyl group.

In some embodiments, one of $R_1$ and $R_2$ may be a substituted or unsubstituted $C_1$-$C_4$ alkyl group, and the other may be a substituted or unsubstituted $C_2$-$C_4$ alkenyl group.

In some embodiments, one of $R_1$ and $R_2$ may be a substituted or unsubstituted methyl group or a substituted or unsubstituted ethyl group, and the other may be a substituted or unsubstituted $C_2$-$C_4$ alkenyl group.

In some embodiments, $R_3$ to $R_6$ may each independently be hydrogen, or a substituted or unsubstituted $C_1$-$C_{10}$ alkyl group.

In some embodiments, $R_3$ to $R_6$ may each independently be hydrogen, or a substituted or unsubstituted $C_1$-$C_6$ alkyl group.

In some embodiments, $R_3$ to $R_6$ may each independently be hydrogen, or a substituted or unsubstituted $C_1$-$C_4$ alkyl group.

In some embodiments, $R_3$ to $R_6$ may each independently be hydrogen, a substituted or unsubstituted methyl group, or a substituted or unsubstituted ethyl group.

In some embodiments, all of $R_3$ to $R_6$ may be hydrogen; one of $R_3$ and $R_4$ may be a substituted or unsubstituted $C_1$-$C_{10}$ alkyl group, and the other may be hydrogen; or one of $R_5$ and $R_6$ may be a substituted or unsubstituted $C_1$-$C_{10}$ alkyl group, and the other may be hydrogen.

In some embodiments, all of $R_3$ to $R_6$ may be hydrogen; one of $R_3$ and $R_4$ may be a substituted or unsubstituted $C_1$-$C_6$ alkyl group, and the other may be hydrogen; or one of $R_5$ and $R_6$ may be a substituted or unsubstituted $C_1$-$C_6$ alkyl group, and the other may be hydrogen.

In some embodiments, all of $R_3$ to $R_6$ may be hydrogen; one of $R_3$ and $R_4$ may be a substituted or unsubstituted $C_1$-$C_4$ alkyl group, and the other may be hydrogen; or one of $R_5$ and $R_6$ may be a substituted or unsubstituted $C_1$-$C_4$ alkyl group, and the other may be hydrogen.

In some embodiments, all of $R_3$ to $R_6$ may be hydrogen; one of $R_3$ and $R_4$ may be a substituted or unsubstituted methyl group, and the other may be hydrogen; or one of $R_5$ and $R_6$ may be a substituted or unsubstituted methyl group, and the other may be hydrogen.

In some embodiments, the butyrolactone-based compound may be represented by any one of Chemical Formulae 2-1 to 2-3.

[Chemical Formula 2-1]

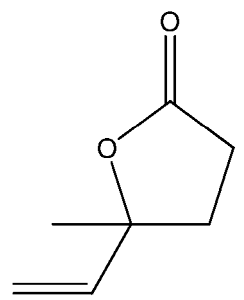

[Chemical Formula 2-2]

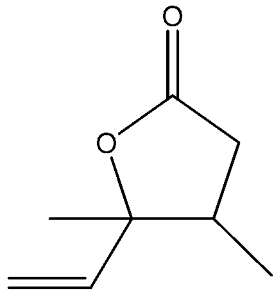

[Chemical Formula 2-3]

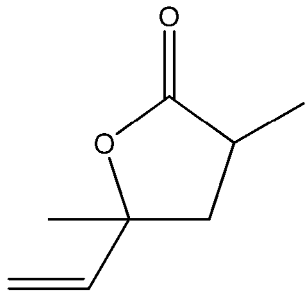

In some embodiments, the butyrolactone-based compound substituted with an alkenyl group may be included in the electrolyte for a lithium secondary battery, a uniform and stable solid electrolyte interphase (SEI) film may be formed on an electrode surface. Accordingly, the compound represented by Chemical Formula 1 may stably protect an active material, thereby improving the high-temperature storage performance of the secondary battery in high-voltage environment. The above effect may be further enhanced when an alkyl group substituent is also includedin addition to the alkenyl group.

For example, the SEI film may stably protect the electrode surface, thereby stabilizing the electrode interface and suppressing side reactions with the electrolyte, so that an increase in the internal resistance of the battery may be suppressed even during high-temperature storage.

For example, the butyrolactone-based compound may form the SEI film having a stable structure even at high temperature, so that the side reactions on the electrode surface may be suppressed, and battery expansion may also be suppressed during charge and discharge.

In some embodiments, a content of the butyrolactone-based compound may be greater than 0 wt% and 10 wt% or less, or from 0.1 wt% to 10 wt% based on a total weight of the electrolyte.

For example, the content of the butyrolactone-based compound may be in a range from 0.2 wt% to 8 wt%, from 0.3 wt% to 5 wt%, from 0.4 wt% to 3 wt%, from 0.5 wt% to 2 wt%, or from 0.1 wt% to 2 wt% based on the total weight of the electrolyte.

In the above ranges, a uniform and stable SEI film may be formed on the electrode surface, and high-temperature storage performance of the lithium secondary battery in high-voltage environments may be provided.

In example embodiments, the electrolyte for a lithium secondary battery may further include at least one auxiliary additive selected from at least one of a cyclic carbonate compound, a fluorine-containing carbonate compound, a lithium phosphate compound, a sultone compound, a borate compound, a sulfate compound, or a sulfite compound.

When the combination of the butyrolactone-based compound and the auxiliary additive is used, the lithium secondary battery having improved low-temperature properties and high-temperature storage properties may be efficiently implemented.

For example, the cyclic carbonate compound may include vinylene carbonate (VC), vinyl ethylene carbonate (VEC), etc.

For example, the fluorine-containing carbonate compound may include a fluorine atom or a fluorine-containing substituent (e.g., a fluorine-substituted alkyl group such as $-CF_3$) bonded to at least one carbon atom of the carbonate compound.

In some embodiments, the fluorine-containing carbonate compound may include a fluorine-containing cyclic carbonate compound having a cyclic structure. For example, the fluorine-containing cyclic carbonate compound may have a 5-7 membered cyclic structure.

For example, the fluorine-containing cyclic carbonate compound may include fluoroethylene carbonate (FEC).

For example, the lithium phosphate compound may include lithium difluoro bis-oxalato phosphate, lithium difluoro phosphate, or others.

In some embodiments, the sultone compound may include at least one selected from an alkyl sultone compound or an alkenyl sultone compound.

In some embodiments, the sultone compound may include both the alkyl sultone compound, the alkenyl sultone compound, etc.

For example, the alkyl sultone compound may include 1,3-propane sultone (PS), 1,4-butane sultone, etc.

For example, the alkenyl sultone compound may include ethene sultone, 1,3-propene sultone (PRS), 1,4-butene sultone, 1-methyl-1,3-propene sultone, etc.

For example, the borate compound may include lithium bis(oxalate) borate.

In some embodiments, the sulfate compound may include a cyclic sulfate compound having a cyclic structure. The cyclic sulfate compound may have a 5-7 membered cyclic structure.

For example, the cyclic sulfate compound may include 1,2-ethylene sulfate (ESA), trimethylene sulfate (TMS), 1,2-propylene sulfate, methyltrimethylene sulfate (MTMS), etc.

In some embodiments, the sulfite compound may include a cyclic sulfite compound having a cyclic structure.

For example, the cyclic sulfite-based compound may include ethylene sulfite, butylene sulfite, etc.

In some embodiments, a content of the auxiliary additive may be in a range from 0.01 wt% to 5 wt% based on the total weight of the electrolyte. For example, the content of the auxiliary additive may be in a range from 0.05 wt% to 9 wt%, from 0.5 wt% to 8 wt%, from 0.8 wt% to 7 wt%, from 1.0 wt% to 6 wt%, from 1.5 wt% to 5 wt%, or from 2 wt% to 4 wt% based on the total weight of the electrolyte.

In the above ranges, durability of the SEI film may be increased without degrading the function of the butyrolactone-based compound. Accordingly, high-temperature storage performance at high voltage of the lithium secondary battery may be further improved.

For example, the secondary battery or the electrolyte may include an organic solvent. The organic solvent may include an organic compound that may have sufficient solubility for a lithium salt, the additive, and the auxiliary additive, and may not be reactive within the lithium secondary battery. In this case, the electrolyte may be provided as a non-aqueous electrolyte solution including the organic solvent

In some embodiments, the organic solvent may be included in an amount of at least 50 wt%, at least 60 wt%, or at least 70 wt% based on the total weight of the electrolyte.

In some embodiments, the organic solvent may include at least one of a carbonate solvent, an ester solvent, an ether solvent, a ketone solvent, an alcohol solvent, or an aprotic solvent.

In some embodiments, the organic solvent may include a carbonate solvent, and the carbonate solvent may include a linear carbonate solvent and/or a cyclic carbonate solvent.

For example, the linear carbonate solvent may include dimethyl carbonate (DMC), ethyl methyl carbonate (EMC), diethyl carbonate (DEC), methyl propyl carbonate, ethyl propyl carbonate, dipropyl carbonate, or others.

For example, the cyclic carbonate solvent may include ethylene carbonate (EC), propylene carbonate (PC), butylene carbonate, or others.

In some embodiments, a volume-based content of the linear carbonate solvent may be greater than that of the cyclic carbonate solvent in the organic solvent.

In some embodiments, a ratio of a volume of the cyclic carbonate solvent to a volume of the linear carbonate solvent in the organic solvent may be in a range from 1/9 to 1. For example, the volume ratio may be in a range from 1/9 to 2/3, from 1/6 to 2/3, or from 1/4 to 2/3. In the above ranges, high-temperature storage properties and low-temperature properties of the lithium secondary battery may be further improved.

For example, the ester solvent may include at least one of methyl acetate (MA), ethyl acetate (EA), n-propyl acetate (n-PA), 1,1-dimethylethyl acetate (DMEA), methyl propionate (MP), ethyl propionate (EP), gamma-butyrolactone (GBL), decanolide, valerolactone, mevalonolactone, or caprolactone.

For example, the ether solvent may include at least one of dibutyl ether, tetraethylene glycol dimethyl ether (TEGDME), diethylene glycol dimethyl ether (DEGDME), dimethoxyethane, tetrahydrofuran (THF), or 2-methyltetrahydrofuran.

For example, the ketone solvent may include cyclohexanone.

For example, the alcohol solvent may include at least one of ethyl alcohol or isopropyl alcohol.

For example, the aprotic solvent may include at least one of a nitrile-based solvent, an amide-based solvent (e.g., dimethylformamide), a dioxolane-based solvent (e.g., 1,3-dioxolane), a sulfolane-based solvent, or others.

In some embodiments, the electrolyte may include a lithium salt.

The lithium salt may be expressed as $Li^+X^-$, and examples of the anion ($X^-$) thereof include $F^-$, $Cl^-$, $Br^-$, $I^-$, $NO_3^-$, $N(CN)_2$, $BF_4^-$, $ClO_4^-$, $PF_6^-$, $(CF_3)_2PF_4^-$, $(CF_3)_3PF_3^-$, $(CF_3)_4PF_2^-$, $(CF_3)_5PF^-$, $(CF_3)_6P^-$, $CF_3SO_3^-$, $CF_3CF_2SO_3^-$, $(CF_3SO_2)_2N^-$, $(FSO_2)_2N^-$, $CF_3CF_2(CF_3)_2CO^-$, $(CF_3SO_2)_2CH^-$, $(SF_5)_3C^-$, $(CF_3SO_2)_3C^-$, $CF_3(CF_2)_7SO_3^-$, $CF_3CO_2^-$, $CH_3CO_2^-$, $SCN^-$, $(CF_3CF_2SO_2)_2N^-$, or others.

In some embodiments, the lithium salt may include at least one selected from $LiPF_6$, $LiClO_4$, $LiBF_4$, LiFSI, LiTFSI, $LiSO_3CF_3$, LiBOB, LiFOB, LiDFOB, LiDFBP, LiTFOP, $LiPO_2F_2$, LiCl, LiBr, LiI, $LiB_{10}Cl_{10}$, $LiCF_3SO_3$, $LiCF_3CO_2$, $LiAsF_6$, $LiSbF_6$, $LiAlCl_4$, $CH_3SO_3Li$, $CF_3SO_3Li$, LiSCN or $LiC(CF_3SO_2)_3$.

In an embodiment, the lithium salt may include at least one selected from $LiPF_6$, LiFSI or LiTFSI.

In some embodiments, the lithium salt may be included at a concentration from 0.01 M to 5 M, from 0.01 M to 4 M, from 0.5 M to 3 M, or from 0.5 M to 2 M in the organic solvent. In the above concentration ranges, transfer of lithium ions and/or electrons may be facilitated during charging and discharging of the lithium secondary battery.

FIGS. 1 and 2 are a schematic plan view and a schematic cross-sectional view, respectively, illustrating a lithium secondary battery in accordance with example embodiments. FIG. 2 is a cross-sectional view taken along line a I-I' of FIG. 1.

Referring to FIGS. 1 and 2, the lithium secondary battery may include an electrode assembly 150 including a cathode 100 and an anode 130, and the electrode assembly may further include a separator 140, a solid electrolyte layer, or a semi-solid electrolyte layer interposed between the cathode and the anode.

For example, the electrode assembly 150 may include the repeatedly stacked cathodes 100 and anodes 130, and the electrode assembly 150 may be accommodated in a case 160 and impregnated with the electrolyte according to embodiments as described above.

The cathode 100 may include a cathode current collector 105, and a cathode active material layer 110 disposed on at least one surface of the cathode current collector 105.

For example, the cathode current collector 105 may include stainless steel, nickel, aluminum, titanium, or an alloy thereof. The cathode current collector may include aluminum or stainless steel that may be surface-treated with carbon, nickel, titanium, or silver. A thickness of the cathode current collector may be, e.g., 10 $\mu$m to 50 $\mu$m, but is not limited thereto.

For example, a cathode active material may include a compound capable of reversibly intercalating and de-intercalating lithium ions.

In some embodiments, the cathode 100 may include a cathode active material including a lithium metal oxide. The lithium metal oxide may further include at least one of nickel (Ni), cobalt (Co), manganese (Mn) or aluminum (Al).

In example embodiments, a nickel content among elements excluding lithium and oxygen in the lithium metal oxide may be in a range from 50 mol% to70 mol%. In some embodiments, the nickel content among elements excluding lithium and oxygen in the lithium metal oxide may be in a range from 55 mol% to 65 mol%.

In the above ranges, a cathode having improved stability may be provided without degrading the capacity of the cathode.

If the content of nickel exceeds 70 mol%, stability of the crystal structure of the lithium metal oxide may be lowered during charge and discharge of the battery, reducing life-span properties of the battery.

If the content of nickel is less than 50 mol%, the capacity of the cathode may be reduced, and the energy efficiency of the battery may also be reduced.

As used herein, "content" may be based on a total moles of each metal contained in a bulk composition of an entire particle of the lithium metal oxide unless otherwise defined.

In some embodiments, the cathode active material may include lithium metal oxide particles having a structure represented by Chemical Formula 3.

[Chemical Formula 3] $Li_xNi_aM_bO_{2+z}$

In Chemical Formula 3, $0.9 \le x \le 1.2$, $0.5 \le a \le 0.9$, $0.01 \le b \le 0.4$, and $-0.5 \le z \le 0.1$. As described above, M may include Co, Mn and/or Al.

The chemical structure represented by Chemical Formula 3 may represent bonding relationship included in a layered structure or a crystal structure included in the cathode active material, and does not exclude other additional elements. For example, in Chemical Formula 3, M may include Co and/or Mn, and Co and/or Mn may serve as main active element of the cathode active material together with Ni. Chemical Formula 3 is provided to express the bonding relationship of the main active elements, and is to be understood as a formula encompassing introduction and substitution of the additional elements.

In an embodiment, an auxiliary element may be further included in addition to the main active elements to enhance chemical stability of the cathode active material or the layered/crystal structure. The auxiliary element may be incorporated in the layered structure/crystal structure to form a bond, and this case is to be understood as being included in the chemical structure of Chemical Formula 3.

The auxiliary element may include at least one selected from e.g., Na, Mg, Ca, Y, Ti, Hf, V, Nb, Ta, Cr, Mo, W, Fe, Cu, Ag, Zn, B, Al, Ga, C, Si, Sn, Sr, Ba, Ra, P or Zr. The auxiliary element may act as an auxiliary active element which may contribute to capacity/power activity of the cathode active material.

For example, the lithium metal oxide may include a layered structure or a crystal structure represented by

Chemical Formula 3-1.

[Chemical Formula 3-1] $Li_xNi_aM1_{b1}M2_{b2}O_{2+z}$

In Chemical Formula 3-1, M1 may include Co, Mn and/or Al. M2 may include the above-mentioned auxiliary element. In Chemical Formula 3-1, $0.9 \leq x \leq 1.2$, $0.5 \leq a \leq 0.9$, $0.01 \leq b1+b2 \leq 0.5$, and $-0.5 \leq z \leq 0.1$.

In some embodiments, the lithium metal oxide may further include a coating element or a doping element. For example, elements substantially the same as or similar to the auxiliary elements described above may be used as the coating element or the doping element. For example, the above elements may be used alone or in a combination of two or more therefrom as the coating element or the doping element.

The coating element or the doping element may be present on a surface of the lithium metal oxide particle, or may penetrate through the surface of the lithium metal oxide particle to be included in the bonding structure represented by Chemical Formula 3 or Chemical Formula 3-1.

The lithium metal oxide particles may have a single particle structure or a secondary particle structure.

In some embodiments, the lithium metal oxide particles may have the single particle structure. As used herein, the term "single particle structure" is intended to exclude the secondary particle formed by agglomeration of multiple primary particles (e.g., more than 10 particles) into a single particle.

For example, the lithium metal oxide particles may substantially consist of particles of the single particle structure or the single particle shape, and the secondary particle structure formed by aggregation or agglomeration of the primary particles may be excluded. The term "single particle structure" as used herein does not exclude, e.g., a single-particle structure in which 2 to 10 single particles are attached or adhered to each other to form a single body or a monolithic structure.

The single particle structure may also include a structure in which multiple primary particles are merged together and transformed into a substantially single particle.

In an embodiment, the lithium metal oxide particles may have the single particle structure having a single crystalline structure or a polycrystalline structure in a crystallographic aspect.

For example, if the single particle has the single crystalline structure, the single particle may substantially consist of one single crystal. If the single particle has the polycrystalline structure, the single particle may include two or more single crystals.

For example, the single crystalline structure and the polycrystalline structure may be distinguished based on an ion image obtained by analyzing a particle cross-section using a Focused Ion Beam (FIB). For example, if a particle has the polycrystalline particle structure, two or more single crystals may be observed in the FIB analysis image based on differences in crystal orientation. For example, even though a particle is observed as a single particle in a Scanning Electron Microscope (SEM) cross-sectional image, the particle may be observed as including two or more crystals in the FIB analysis image.

In example embodiments, in the crystal orientation analysis of the cross-sectional images of the lithium metal oxide particles obtained using the FIB, an average value of particle diameters in a major axis direction may be 1 μm or greater. For example, in the crystal orientation analysis of the cross-sectional images of 10 or more lithium metal oxide particles obtained using the FIB, the average value of the particle diameters in the major axis direction may be in a range from 1 μm to 5 μm.

For example, a cathode slurry may be prepared by mixing the cathode active material in a solvent. The cathode slurry can be coated on the cathode current collector, and then dried and pressed to form the cathode active material layer 110.

The coating process may be performed using a method such as a gravure coating, a slot die coating, a multi-layer simultaneous die coating, an imprinting, a doctor blade coating, a dip coating, a bar coating, a casting, etc., but is not limited thereto.

The cathode active material layer 110 may further include a binder, and may optionally include a conductive material, a thickener, etc.

Non-limiting examples of the solvent used in the preparation of the cathode active material layer 110 include N-methyl-2-pyrrolidone (NMP), dimethylformamide, dimethylacetamide, N,N-dimethylaminopropylamine, ethylene oxide, tetrahydrofuran, etc.

The binder may include polyvinylidene fluoride (PVDF), vinylidene fluoride-cohexafluoropropylene copolymer, polyacrylonitrile, polymethylmethacrylate, acrylonitrile butadiene rubber (NBR), poly(butadiene) rubber (BR), styrene-butadiene rubber (SBR), etc. In an embodiment, a PVDF-based binder may be used as the cathode binder.

The conductive material may be added to enhance the conductivity of the cathode active material layer 110 and/or mobility of lithium ions or electrons. For example, the conductive material may include a carbon-based conductive material such as graphite, carbon black, acetylene black, Ketjen black, graphene, carbon nanotubes, a vapor-grown carbon fiber (VGCF), a carbon fiber, etc., and/or a metal-based conductive material such as tin, tin oxide, titanium oxide, a

perovskite material such as $LaSrCoO_3$ and $LaSrMnO_3$, etc.

The cathode active material layer 110 may further include a thickener and/or a dispersant. For example, the cathode active material layer 110 may include a thickener such as carboxymethyl cellulose (CMC).

The anode 130 may include an anode current collector 125, and an anode active material layer 120 disposed on at least one surface of the anode current collector 125.

For example, non-limiting examples of the anode current collector 125 include a copper foil, a nickel foil, a stainless steel foil, a titanium foil, a nickel foam, a copper foam, a polymer substrate coated with a conductive metal, etc. A thickness of the anode current collector may be in a range from 10 μm to 50 μm, but is not limited thereto.

The anode active material layer 120 may include an anode active material. A material capable of adsorbing and desorbing lithium ions may be used as the anode active material. For example, the anode active material may be a carbon-based material such as a crystalline carbon, an amorphous carbon, a carbon composite, a carbon fiber, etc.; a lithium metal; a lithium alloy; a silicon (Si)-containing material, or a tin (Sn)-containing material, etc.

Examples of the amorphous carbon include hard carbon, soft carbon, coke, a mesocarbon microbead (MCMB), a mesophase pitch-based carbon fiber (MPCF), etc.

Examples of the crystalline carbon include a graphite-based carbon such as natural graphite, artificial graphite, a graphitized coke, a graphitized MCMB, a graphitized MPCF, etc.

The lithium metal may be pure lithium metal or a lithium metal with a protective layer formed thereon to suppress dendrite growth. In an embodiment, a lithium metalcontaining layer deposited or coated on the anode current collector 125 may be used as the anode active material layer 120. In an embodiment, a lithium thin- film layer may also be used as the anode active material layer 120.

Elements included in the lithium alloy may include aluminum, zinc, bismuth, cadmium, antimony, silicon, lead, tin, gallium, indium, etc.

The silicon-containing material may provide increased capacity properties. The silicon-containing material may include Si, $SiO_x$ ($0<x<2$), a metal-doped $SiO_x$ ($0<x<2$), a silicon-carbon composite, etc. The metal may include lithium and/or magnesium, and the metal-doped $SiO_x$ ($0<x<2$) may include a metal silicate.

For example, the anode active material may be mixed in a solvent to prepare an anode slurry. The anode slurry may be coated/deposited on the anode current collector 125, and then dried and pressed to form the anode active material layer 120.

The coating process may be performed using a method such as a gravure coating, a slot die coating, a multi-layer simultaneous die coating, an imprinting, a doctor blade coating, a dip coating, a bar coating, a casting, etc., but is not limited thereto.

The anode active material layer 120 may further include a binder, and may optionally further include a conductive material, a thickener, etc.

In some embodiments, the anode 130 may include the anode active material layer 120 in the form of a lithium metal formed by a deposition/coating process.

Non-limiting examples of the solvent for the anode active material layer 120 include water, pure water, deionized water, distilled water, ethanol, isopropanol, methanol, acetone, n-propanol, t-butanol, or others.

The aforementioned materials that may be used in the formation of the cathode may also be used as the binder, the conductive material, and the thickener for the formation of the anode.

In some embodiments, the anode binder may include a styrene-butadiene rubber (SBR)-based binder, carboxymethyl cellulose (CMC), a polyacrylic acid-based binder, a poly(3,4-ethylenedioxythiophene) (PEDOT)-based binder, ect.

The separator 140 may be interposed between the cathode 100 and the anode 130. The separator 140 may prevent electrical short-circuit between the cathode and the anode while maintaining the flow of ions between the anode and cathode. For example, a thickness of the separator 140 may be in a range from 10 μm to 20 μm, but is not limited thereto.

For example, the separator 140 may include a porous polymer film or a porous nonwoven fabric. The porous polymer film may include a polyolefin-based polymer such as an ethylene polymer, a propylene polymer, an ethylene/-butene copolymer, an ethylene/hexene copolymer, an ethylene/methacrylate copolymer, or the like. The porous non-woven fabric may include a high-melting-point glass fiber, a polyethylene terephthalate fiber, or others.

The separator 140 may also include a ceramic material. For example, inorganic particles may be coated on the polymer film or dispersed in the polymer film to improve heat resistance.

The separator 140 may have a single-layered structure or a multi-layered structure including the aforementioned polymer film and/or non-woven fabric.

In example embodiments, the cathode 100, the anode 130, and the separator 140 may be repeatedly arranged to form the electrode assembly 150. In some embodiments, the form of the electrode assembly 150 may be a winding type, a stacking type, a z-folding type, a stackfolding type, or others.

The electrode assembly 150 may be accommodated in the case together with the electrolyte according to the

above-described embodiments to provide the lithium secondary battery.

For example, electrode tabs (a cathode tab and an anode tab) included in each electrode cell may protrude from the cathode current collector 105 and the anode current collector 125, respectively, to extend to one side of the case 160. The electrode tabs may be fused together with the one side of the case 160 to be connected to an electrode lead (a cathode lead 107 and an anode lead 127) which are extended or exposed to an outside of the case 160.

For example, a pouch-shaped case, a prismatic case, a cylindrical case, a coin-shaped case, etc., may be used as the case 160.

The lithium secondary battery may have an operating voltage in a range from 4.3 V to 4.7 V. For example, the lithium secondary battery may have the operating voltage in a range from 4.35 V to 4.5 V. Accordingly, the battery may be operated in a high voltage range.

The invention relates also to the following numbered aspects:

Aspect 1. An electrolyte for a lithium secondary battery, comprising:

a butyrolactone-based compound comprising at least one alkenyl group; and
a lithium salt.

Aspect 2. The electrolyte for a lithium secondary battery of aspect 1, wherein the butyrolactone-based compound includes a gamma-butyrolactone-based compound.

Aspect 3. The electrolyte for a lithium secondary battery of aspect 1 or 2, wherein the butyrolactone-based compound further comprises at least one alkyl group.

Aspect 4. The electrolyte for a lithium secondary battery of any one of aspects 1 to 3, wherein the butyrolactone-based compound is represented by Chemical Formula 1:

[Chemical Formula 1]

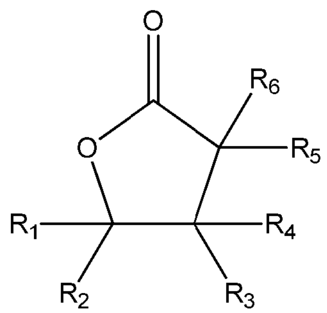

wherein, in Chemical Formula 1, $R_1$ to $R_6$ are each independently hydrogen, a $C_1$-$C_{10}$ substituted or unsubstituted alkyl group, or a $C_2$-$C_{10}$ substituted or unsubstituted alkenyl group, and at least one of $R_1$ to $R_6$ is a $C_2$-$C_{10}$ substituted or unsubstituted alkenyl group.

Aspect 5. The electrolyte for a lithium secondary battery of aspect 4, wherein at least one of $R_1$ to $R_6$ is a $C_1$-$C_6$ substituted or unsubstituted alkyl group.

Aspect 6. The electrolyte for a lithium secondary battery of aspect 4 or 5, wherein one or two of $R_1$ to $R_6$ is a substituted or unsubstituted $C_1$-$C_6$ alkyl group, and one of remaining groups is a substituted or unsubstituted $C_2$-$C_{10}$ alkenyl group.

Aspect 7. The electrolyte for a lithium secondary battery of any one of aspects 4 to 6, wherein (a) $R_1$ to $R_6$ are each independently hydrogen, a substituted or unsubstituted $C_1$-$C_6$ alkyl group, or a substituted or unsubstituted $C_2$-$C_6$ alkenyl group, and at least one of $R_1$ to $R_6$ is a substituted or unsubstituted $C_2$-$C_6$ alkenyl group, or
(b) $R_1$ to $R_6$ are each independently hydrogen, a $C_1$-$C_4$ substituted or unsubstituted alkyl group, or a $C_2$-$C_4$ substituted or unsubstituted alkenyl group, and at least one of $R_1$ to $R_6$ is a $C_2$-$C_4$ substituted or unsubstituted alkenyl group.

Aspect 8. The electrolyte for a lithium secondary battery of any one of aspects 1 to 7, wherein the butyrolactone-based compound comprises a compound represented by any one of Chemical Formulae 2-1 to 2-3:

[Chemical Formula 2-1]

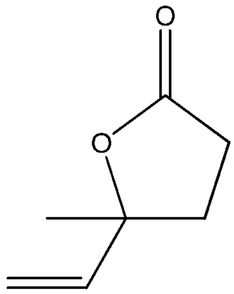

[Chemical Formula 2-2]

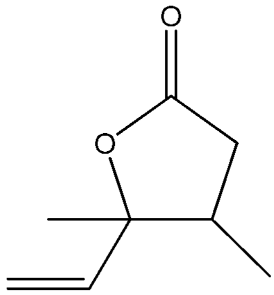

[Chemical Formula 2-3]

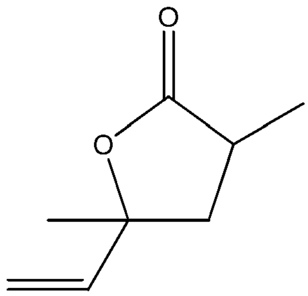

Aspect 9. The electrolyte for a lithium secondary battery of any one of aspects 1 to 8, wherein a content of the butyrolactone-based compound is greater than 0 wt%, and 10 wt% or less based on a total weight of the electrolyte.
Aspect 10. The electrolyte for a lithium secondary battery of any one of aspects 1 to 9, further comprising an organic solvent, wherein the organic solvent comprises at least one of a carbonate solvent, an ester solvent, an ether solvent, a ketone solvent, an alcohol solvent or an aprotic solvent.
Aspect 11. The electrolyte for a lithium secondary battery of any one of aspects 1 to 10, further comprising at least one auxiliary additive selected from a cyclic carbonate compound, a fluorine-containing carbonate compound, a lithium phosphate compound, a sultone compound, a borate compound, a sulfate compound or a sulfite compound, and preferably a content of the auxiliary additive is in a range from 0.01 wt% to 5 wt% based on a total weight of the electrolyte.
Aspect 12. A lithium secondary battery, comprising:

an electrode assembly comprising a cathode and an anode; and
the electrolyte for a lithium secondary battery of any one of aspects 1 to 11.

Aspect 13. The lithium secondary battery of aspect 12, wherein the cathode comprises a cathode active material that includes lithium metal oxide particles having a single particle structure, and preferably a content of nickel in the lithium metal oxide particles is in a range from 50 mol% to 70 mol% based on total moles of elements excluding lithium and oxygen contained in the lithium metal oxide particles.
Aspect 14. The lithium secondary battery of aspect 12 or 13, wherein the lithium secondary battery has an operating voltage of 4.35 V to 4.5 V.

Aspect 15. The lithium secondary battery of any one of aspects 12 to 14, wherein the anode comprises a solid electrolyte interphase (SEI) film on a surface thereof, and the SEI film is formed from the electrolyte.

EXAMPLE 1

Synthesis Example 1 (Synthesis of Chemical Formula 2-1):5-ethenyldihydro-5-methyl-furan-2-one:

Ethyl levulinate (8.5 mL, 60 mmol) and dichloromethane (120 mL) were sequentially added to a round-bottom flask. The mixture was cooled to 0°C and stirred. 1 M vinyl magnesium bromide (66 mL, 66 mmol) was slowly added dropwise to the mixture over 1 hour while maintaining a temperature at 0°C, and the temperature was increased to room temperature while stirring for 3 hours.

Thereafter, the product was washed with a saturated aqueous ammonium chloride solution and distilled water, and a solvent in a remaining organic layer was removed under reduced pressure. The dried product was purified by a silica gel column chromatography and solvent-dried to yield 6 g of a product (Chemical Formula 2-1) as a colorless liquid.

[Chemical Formula 2-1]

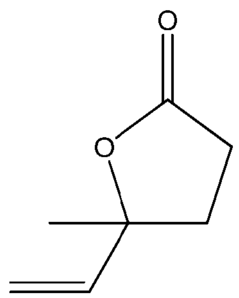

Preparation of electrolyte:

A 1.0 M $LiPF_6$ solution (including a ethylene carbonate (EC)/ethyl methyl carbonate (EMC) mixed solvent in a volume ratio of 20:80) was prepared.

Based on a total weight of the electrolyte (100 wt%), 1.5 wt% of fluoroethylene carbonate (FEC), 1.0 wt% of W3, 0.5 wt% of PS, 0.3 wt% of PRS, and 0.5 wt% of ESA were added to the $LiPF_6$ solution, and 0.3 wt% of an additive represented by Chemical Formula 2-1, to prepare the electrolyte.

Fabrication of lithium secondary battery:

A slurry was prepared by mixing $LiNi_{0.6}Co_{0.1}Mn_{0.3}O_2$ of a single particle structure (particle size: 3.5 μm) as a cathode active material, carbon black as a conductive material, and polyvinylidene fluoride (PVDF) as a binder in a weight ratio of 92:5:3. The slurry was uniformly coated on an aluminum foil (thickness: 15 μm), vacuum-dried at 130°C, and pressed to produce a cathode for a lithium secondary battery.

An anode slurry containing 95 wt% of an anode active material (a mixture of artificial graphite and natural graphite in a weight ratio of 7:3), 1 wt% of Super-P as a conductive material, 2 wt% of styrene-butadiene rubber (SBR) as a binder, and 2 wt% of carboxymethyl cellulose (CMC) as a thickener was prepared. The anode slurry was uniformly coated on a copper foil (thickness: 15 μm), and then dried and pressed to form an anode.

The cathodes and the anodes prepared as described above were each cut to a predetermined size, and stacked with a separator (polyethylene, thickness: 20 μm) interposed therebetween to form an electrode assembly. The tab portions of the cathodes and the anodes were each welded.

The electrode assembly was placed in a pouch and sealed at three sides except for an electrolyte injection side. An area around the electrode tabs was included in the sealing portion. The electrolyte manufactured in the above (1) was injected through the electrolyte injection side, and then the electrolyte injection side was also sealed. Subsequently, impregnation was performed for more than 12 hours to obtain a lithium secondary battery.

EXAMPLE 2

Synthesis Example 2 (Synthesis of Chemical Formula 2-2): 4,5-dimethyl-5-vinyl-dihydro-furan-2-one:

Ethyl 3-methyl-4-oxopentanoate (9.7 mL, 60 mmol) and dichloromethane (120 mL) were sequentially added to a round-bottom flask. The mixture was cooled to 0°C and stirred. 1 M vinyl magnesium bromide (66 mL, 66 mmol) was slowly added dropwise to the mixture over 1 hour while maintaining the temperature at 0°C, and the temperature was increased to room temperature while stirring for 3 hours.

Thereafter, the product was washed with a saturated aqueous ammonium chloride solution and distilled water, and a solvent in a remaining organic layer was removed under reduced pressure. The dried product was purified by a silica gel column chromatography and solvent-dried to obtain 6 g of a product (Chemical Formula 2-2) in the form of a colorless liquid.

[Chemical Formula 2-2]

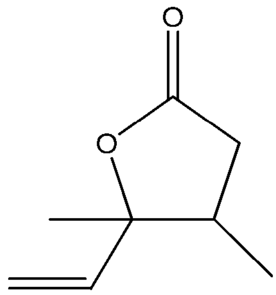

An electrolyte and a lithium secondary battery sample were prepared by the same method as that in Example 1, except that 0.3 wt% of an additive represented by Chemical Formula 2-2 was used instead of the additive in Example 1.

EXAMPLE 3

Synthesis Example 3 (Synthesis of Chemical Formula 2-3): 3,5-dimethyl-5-vinyl-dihydro-furan-2-one:

Ethyl 2-methyl-4-oxopentanoate (9.6 mL, 60 mmol) and dichloromethane (120 mL) were sequentially added to a round-bottom flask. The mixture was cooled to 0°C and stirred. 1 M vinyl magnesium bromide (66 mL, 66 mmol) was slowly added dropwise to the mixture over 1 hour, and the temperature was increased to room temperature while stirring for 3 hours.

Thereafter, the product was washed with a saturated aqueous ammonium chloride solution and distilled water, and a solvent in a remaining organic layer was removed under reduced pressure. The dried product was purified by a silica gel column chromatography and solvent-dried to obtain 6 g of a product (Chemical Formula 2-3) in the form of a colorless liquid.

[Chemical Formula 2-3]

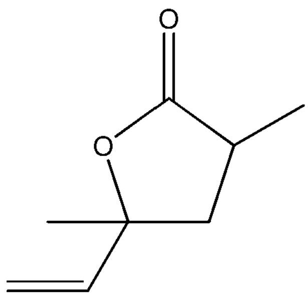

An electrolyte and a lithium secondary battery sample were prepared by the same method as that in Example 1, except that 0.3 wt% of an additive represented by Chemical Formula 2-3 was used instead of the additive in Example 1.

EXAMPLE 4

An electrolyte and a lithium secondary battery sample were prepared by the same method as in Example 1,

except that 0.5 wt% of the additive in Example 1 was used.

EXAMPLE 5

An electrolyte and a lithium secondary battery sample were prepared by the same method as that in Example 1, except that 0.75 wt% of the additive in Example 1 was used.

EXAMPLE 6

An electrolyte and a lithium secondary battery sample were prepared by the same method as that in Example 1, except that 1.0 wt% of the additive of Example 1 was used.

COMPARATIVE EXAMPLE 1

An electrolyte and a lithium secondary battery sample were prepared by the same method as that in Example 1, except that the additive of Example 1 was not added.

COMPARATIVE EXAMPLE 2

An electrolyte and a lithium secondary battery sample were prepared by the same method as that in Example 1, except that 0.3 wt% of an additive represented by Chemical Formula 2-4 was used instead of the additive of Example 1.

[Chemical Formula 2-4]

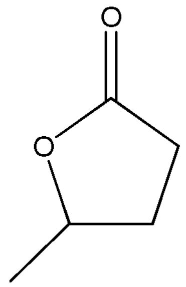

The compositions of the electrolyte in the Examples and Comparative Examples are shown in Table 1 below.

TABLE 1

| | Lithium salt | Additive (wt%) | Auxiliary additive (wt%) | | | | |
|---|---|---|---|---|---|---|---|
| | | | FEC | W3 | PS | PRS | ESA |
| Example 1 | $LiPF_6$ (1.0M) | Additive 1 (0.3) | 1.5 | 1.0 | 0.5 | 0.3 | 0.5 |
| Example 2 | $LiPF_6$ (1.0M) | Additive 2 (0.3) | 1.5 | 1.0 | 0.5 | 0.3 | 0.5 |
| Example 3 | $LiPF_6$ (1.0M) | Additive 3 (0.3) | 1.5 | 1.0 | 0.5 | 0.3 | 0.5 |
| Example 4 | $LiPF_6$ (1.0M) | Additive 1 (0.5) | 1.5 | 1.0 | 0.5 | 0.3 | 0.5 |
| Example 5 | $LiPF_6$ (1.0M) | Additive 1 (0.75) | 1.5 | 1.0 | 0.5 | 0.3 | 0.5 |
| Example 6 | $LiPF_6$ (1.0M) | Additive 1 (1.0) | 1.5 | 1.0 | 0.5 | 0.3 | 0.5 |
| Comparative Example 1 | $LiPF_6$ (1.0M) | - | 1.5 | 1.0 | 0.5 | 0.3 | 0.5 |
| Comparative Example 2 | $LiPF_6$ (1.0M) | Additive 4 (0.3) | 1.5 | 1.0 | 0.5 | 0.3 | 0.5 |

The components listed in Table 1 are as follows.

Additive 1: the compound represented by Chemical Formula 2-1
Additive 2: the compound represented by Chemical Formula 2-2
Additive 3: the compound represented by Chemical Formula 2-3

Additive 4: the compound represented by Chemical Formula 2-4
FEC: fluoroethylene carbonate
W3: lithium difluorophosphate
PS: propanesultone
PRS: propenesultone
ESA: ethylene sulfate

A battery performance was evaluated as follows, and the results are shown in Tables 2 and 3.

Initial Performance Evaluation:

(1) Evaluation of initial discharge capacity

The lithium secondary batteries of Examples and Comparative Examples were charged at 25 °C under conditions of 0.5 C-rate, CC/CV (4.35 V, 0.05 C cut-off), and discharged under conditions of 0.5 C-rate, CC (2.7 V cutoff), and 3 cycles of the charge and discharge were performed.
A discharge capacity value at the third cycle was defined as an initial discharge capacity (C1) of the lithium secondary battery.

(2) Evaluation of initial resistance

For the lithium secondary batteries of the Examples and Comparative Examples, while sequentially increasing a C-rate from 0.2C, 0.5C, 1.0C, 1.5C, 2.0C, 2.5C and 3.0C at a 60% state of charge (SOC), charge and discharge were performed at each C-rate for 10 seconds. A linear equation was set using terminal voltage points, and a slope was adopted as a DCIR.

High-temperature Storage Performance Evaluation (60°C):

(1) Evaluation of capacity retention

The lithium secondary batteries of Examples and Comparative Examples were charged under conditions of 0.2 C-rate, CC/CV (4.35 V, 0.05C cut-off) at 25°C and then stored in a constant temperature and humidity chamber at 60°C for 6 weeks.
The lithium secondary batteries of Examples and Comparative Examples stored at 60°C for 6 weeks were subjected to 0.5 C-rate CC discharge (2.7 V cut-off), and a discharge capacity (C2) after the high-temperature storage was measured.
A capacity retention was calculated as follows:

$$\text{Capacity retention (\%)} = (C2/C1) \times 100\ (\%)$$

(2) Evaluation of capacity recovery

After measuring the capacity retention of the lithium secondary batteries of Examples and Comparative Examples as described in the above (1), a charging at 0.5 C-rate CC/CV (4.35 V, 0.05 C cut-off) and discharging at 0.5 C-rate CC (2.5 V cut-off) were performed, and then a discharge capacity (C3) was measured.
A capacity recovery was calculated as follows:

$$\text{Capacity recovery (\%)} = (C3/C1) \times 100\ (\%)$$

(3) Evaluation of resistance increase ratio

The lithium secondary batteries of Examples and Comparative Examples were charged at 0.2 C-rate CC/CV (4.35 V, 0.05 C cutoff) at 25°C and then stored in a constant temperature and humidity chamber at 60°C for 6 weeks.
At a 60% SOC, while sequentially increasing a C-rate from 0.2C, 0.5C, 1.0C, 1.5C, 2.0C, 2.5C and 3.0C at a 60%

state of charge (SOC), charge and discharge were performed at each C-rate for 10 seconds. A linear equation was set using terminal voltage points, and a slope was adopted as a DCIR.

A high-temperature internal resistance (DCIR) measured from the lithium secondary battery of Comparative Example 1 was set as 100, and the high-temperature internal resistance (DCIR) measured from each lithium secondary battery Examples and Comparative Examples was scaled accordingly.

(4) Evaluation of thickness increase ratio

Each lithium secondary battery of the Examples and Comparative Examples were charged under conditions of 0.5C CC/CV (4.35V 0.05C CUT-OFF) at 25°C, and then a battery thickness (T1) was measured. The charged lithium secondary battery was exposed to air at 60°C for 6 weeks (using a constant-temperature device), and then a battery thickness (T2) was measured.

The battery thickness was measured using a flat plate thickness measuring device (Mitutoyo, 543-490B). A battery thickness increase ratio was calculated as follows:

$$\text{Battery thickness increase ratio (\%)} = T2/T1 \times 100\ (\%)$$

TABLE 2

| | | Example 1 | Example 2 | Example 3 | Example 4 | Example 5 | Example 6 |
|---|---|---|---|---|---|---|---|
| Initial performance | Capacity (mAh) | 1899 | 1887 | 1896 | 1899 | 1864 | 1882 |
| | Resistance (DCIR) (mΩ) | 43.6 | 44.2 | 44.6 | 45.1 | 46.2 | 48.3 |
| 60°C High temperature storage performance (6 weeks) | Capacity retention (%) | 83.2 | 82.8 | 83.2 | 83.3 | 83.1 | 82.8 |
| | Capacity recovery (%) | 85.6 | 84.6 | 85.1 | 85.4 | 85.3 | 84.9 |
| | Resistance increase ratio (%) | 150.4 | 152.1 | 151.8 | 147.6 | 144.4 | 141.9 |
| | Thickness increase ratio (%) | 103.2 | 103.5 | 105.8 | 103.1 | 102.3 | 102.1 |

TABLE 3

| | | Comparative Example 1 | Comparative Example 2 |
|---|---|---|---|
| Initial performance | Capacity (mAh) | 1880 | 1866 |
| | Resistance (DCIR) (mΩ) | 45.4 | 44.7 |
| 60°C High temperature storage performance (6 weeks) | Capacity retention (%) | 77.0 | 78.5 |
| | Capacity recovery (%) | 79.1 | 80.5 |
| | Resistance increase ratio (%) | 167.4 | 162.2 |
| | Thickness increase ratio (%) | 107.4 | 109.2 |

Referring to Tables 1 to 3, in the lithium secondary batteries of Examples, the initial performance (increased capacity, reduced DCIR) and high-temperature storage performance were entirely enhanced.

In the lithium secondary battery of Comparative Example 1 where the additive including the butyrolactone-based compound with at least one alkenyl group was not included, the initial resistance was increased, and the resistance increase ratio and thickness increase ratio were also increased after the high-temperature storage.

In Comparative Example 2 where the additive containing the butyrolactone-based compound without an alkenyl

group was used, the initial discharge capacity was slightly reduced and the high-temperature storage performance was slightly improved compared to those from Comparative Example 1. However, in Comparative Example 2, the capacity retention and the capacity recovery after the high-temperature storage were reduced, and the resistance increase ratio and thickness increase ratio were increased compared to those from Examples.

## Claims

1. An electrolyte for a lithium secondary battery, comprising:

   a butyrolactone-based compound comprising at least one alkenyl group; and
   a lithium salt.

2. The electrolyte for a lithium secondary battery of claim 1, wherein the butyrolactone-based compound includes a gamma-butyrolactone-based compound.

3. The electrolyte for a lithium secondary battery of claim 1 or 2, wherein the butyrolactone-based compound further comprises at least one alkyl group.

4. The electrolyte for a lithium secondary battery of any one of claims 1 to 3, wherein the butyrolactone-based compound is represented by Chemical Formula 1:

[Chemical Formula 1]

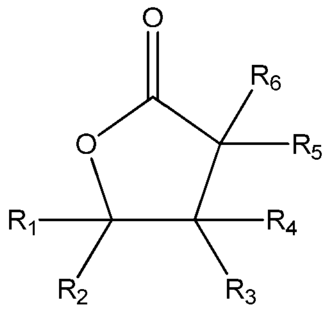

wherein, in Chemical Formula 1, $R_1$ to $R_6$ are each independently hydrogen, a $C_1$-$C_{10}$ substituted or unsubstituted alkyl group, or a $C_2$-$C_{10}$ substituted or unsubstituted alkenyl group, and at least one of $R_1$ to $R_6$ is a $C_2$-$C_{10}$ substituted or unsubstituted alkenyl group.

5. The electrolyte for a lithium secondary battery of claim 4, wherein at least one of $R_1$ to $R_6$ is a $C_1$-$C_6$ substituted or unsubstituted alkyl group.

6. The electrolyte for a lithium secondary battery of claim 4 or 5, wherein one or two of $R_1$ to $R_6$ is a substituted or unsubstituted $C_1$-$C_6$ alkyl group, and one of remaining groups is a substituted or unsubstituted $C_2$-$C_{10}$ alkenyl group.

7. The electrolyte for a lithium secondary battery of any one of claims 4 to 6, wherein (a) $R_1$ to $R_6$ are each independently hydrogen, a substituted or unsubstituted $C_1$-$C_6$ alkyl group, or a substituted or unsubstituted $C_2$-$C_6$ alkenyl group, and at least one of $R_1$ to $R_6$ is a substituted or unsubstituted $C_2$-$C_6$ alkenyl group, or
   (b) $R_1$ to $R_6$ are each independently hydrogen, a $C_1$-$C_4$ substituted or unsubstituted alkyl group, or a $C_2$-$C_4$ substituted or unsubstituted alkenyl group, and at least one of $R_1$ to $R_6$ is a $C_2$-$C_4$ substituted or unsubstituted alkenyl group.

8. The electrolyte for a lithium secondary battery of any one of claims 1 to 7, wherein the butyrolactone-based compound comprises a compound represented by any one of Chemical Formulae 2-1 to 2-3:

[Chemical Formula 2-1]

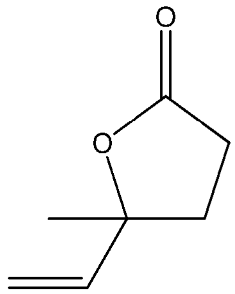

[Chemical Formula 2-2]

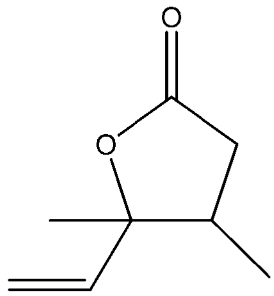

[Chemical Formula 2-3]

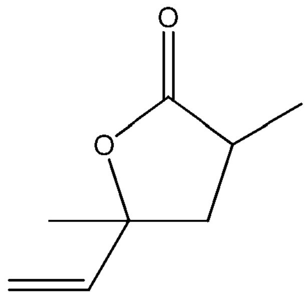

**9.** The electrolyte for a lithium secondary battery of any one of claims 1 to 8, wherein a content of the butyrolactone-based compound is greater than 0 wt%, and 10 wt% or less based on a total weight of the electrolyte.

**10.** The electrolyte for a lithium secondary battery of any one of claims 1 to 9, further comprising an organic solvent, wherein the organic solvent comprises at least one of a carbonate solvent, an ester solvent, an ether solvent, a ketone solvent, an alcohol solvent or an aprotic solvent.

**11.** The electrolyte for a lithium secondary battery of any one of claims 1 to 10, further comprising at least one auxiliary additive selected from a cyclic carbonate compound, a fluorine-containing carbonate compound, a lithium phosphate compound, a sultone compound, a borate compound, a sulfate compound or a sulfite compound, and preferably a content of the auxiliary additive is in a range from 0.01 wt% to 5 wt% based on a total weight of the electrolyte.

**12.** A lithium secondary battery, comprising:

an electrode assembly comprising a cathode and an anode; and
the electrolyte for a lithium secondary battery of any one of claims 1 to 11.

**13.** The lithium secondary battery of claim 12, wherein the cathode comprises a cathode active material that includes lithium metal oxide particles having a single particle structure, and preferably
a content of nickel in the lithium metal oxide particles is in a range from 50 mol% to 70 mol% based on total moles of elements excluding lithium and oxygen contained in the lithium metal oxide particles.

**14.** The lithium secondary battery of claim 12 or 13, wherein the lithium secondary battery has an operating voltage of 4.35 V to 4.5 V.

**15.** The lithium secondary battery of any one of claims 12 to 14, wherein the anode comprises a solid electrolyte interphase (SEI) film on a surface thereof, and the SEI film is formed from the electrolyte.

FIG. 1

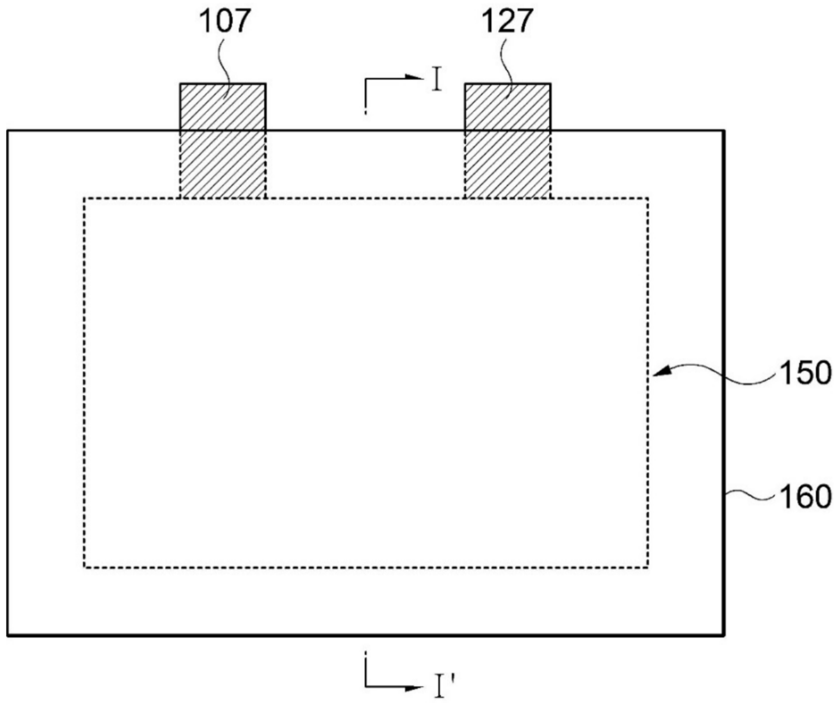


FIG. 2

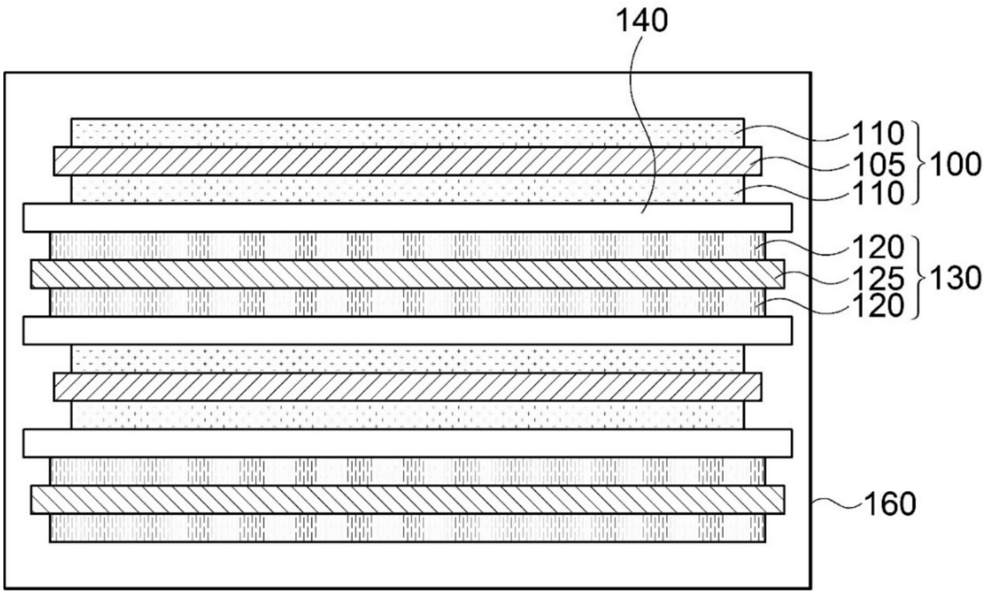

Europäisches Patentamt
European Patent Office
Office européen des brevets

# EUROPEAN SEARCH REPORT

Application Number
EP 25 22 6249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/043482 A1 (LG ENERGY SOLUTION LTD [KR]) 29 February 2024 (2024-02-29)<br>* claims 5, 7, 12 *<br>----- | 1-7,9-15 | INV.<br>H01M10/0567<br>C07D307/00 |

TECHNICAL FIELDS SEARCHED (IPC)
H01M
C07D

The present search report has been drawn up for all claims

1

| Place of search | Date of completion of the search | Examiner |
|---|---|---|
| Munich | 29 April 2026 | Bettio, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 25 22 6249

This annex lists the patent family members relating to the patent documents cited in the above-mentioned European search report. The members are as contained in the European Patent Office EDP file on
The European Patent Office is in no way liable for these particulars which are merely given for the purpose of information.

29-04-2026

| Patent document cited in search report | Publication date | Patent family member(s) | Publication date |
|---|---|---|---|
| WO 2024043482 A1 | 29-02-2024 | CN 118339694 A | 12-07-2024 |
| | | EP 4421936 A1 | 28-08-2024 |
| | | JP 7794528 B2 | 06-01-2026 |
| | | JP 2024542550 A | 15-11-2024 |
| | | KR 20240029301 A | 05-03-2024 |
| | | US 2025343262 A1 | 06-11-2025 |
| | | WO 2024043482 A1 | 29-02-2024 |

EPO FORM P0459

For more details about this annex : see Official Journal of the European Patent Office, No. 12/82